Europäisches Patentamt

European Patent Office

Office européen des brevets

(11) Veröffentlichungsnummer : **0 219 797 B1**

# EUROPÄISCHE PATENTSCHRIFT

(45) Veröffentlichungstag der Patentschrift :
26.04.89

(51) Int. Cl.⁴ : **C 07 D333/28**

(21) Anmeldenummer : 86114182.8

(22) Anmeldetag : 14.10.86

(54) Verfahren zur Herstellung von 2.3-Dichlorthiophen.

(30) Priorität : 19.10.85 DE 3537288

(43) Veröffentlichungstag der Anmeldung :
29.04.87 Patentblatt 87/18

(45) Bekanntmachung des Hinweises auf die Patenterteilung : 26.04.89 Patentblatt 89/17

(84) Benannte Vertragsstaaten :
AT BE CH DE FR GB IT LI NL

(56) Entgegenhaltungen :
EP--A-- 0 166 182
EP--A-- 0 182 111
DE--B-- 1 768 209

(73) Patentinhaber : HOECHST AKTIENGESELLSCHAFT
Postfach 80 03 20
D-6230 Frankfurt am Main 80 (DE)

(72) Erfinder : Litterer, Heinz, Dr.
Hardtstrasse 77
D-6208 Bad Schwalbach (DE)
Erfinder : Leupold, Ernst Ingo, Dr.
Am Zäunefeld 15
D-6392 Neu-Anspach (DE)

## EP 0 219 797 B1

**Beschreibung**

Die vorliegende Erfindung betrifft ein Verfahren zur Herstellung von 2.3-Dichlorthiophen.

Halogenierte Thiophene sind wertvolle Zwischenprodukte zur Herstellung von Pharmazeutika, Pflanzenschutzmitteln und Farbstoffen [Manufacturing Chemist and Aerosol News, May 1978]. So finden unter anderem Derivate der 2.3-Dihalogenthiophene Verwendung als ß-Blocker oder als antiparasitäre Wirkstoffe [Chemiker-Zeitung 103 (5), 161 bis 172 (1979)].

Einer breiteren Anwendung der 2.3-Dihalogenthiophene steht bisher ihre schlechte Zugänglichkeit entgegen.

So kann 2.3-Dichlorthiophen durch Pyrolyse aus dem bei der Chlorierung von Thiophen zu 2-Chlorthiophen mit geringer Selektivität anfallenden optisch inaktiven 2.3.4.5-Tetrachlortetrahydrothiophen im Verhältnis von 1 : 1 zusammen mit 2.4-Dichlorthiophen gewonnen werden. [Coonradt et al., J. Am. Chem. Soc. 70, 2564 (1948)].

Die Umsetzung der ebenfalls sehr teuren Ausgangsstoffe 2.3-Dibromthiophen oder 2.3-Dijodthiophen mit CuCl zu 2.3-Dichlorthiophen ist mehr von wissenschaftlichem Interesse [Conde, S. et al., Synthesis 6, 412 (1976)].

Es wurde nun gefunden, daß 2.3-Dichlorthiophen in guten Ausbeuten durch Isomerisierung von 2-Chlorthiophen mit nachfolgender Chlorierung des dabei anfallenden 3-Chlorthiophens erhalten wird.

Ein Gegenstand der Erfindung ist daher ein Verfahren zur Herstellung von 2.3-Dichlorthiophen, dadurch gekennzeichnet, daß man 2-Chlorthiophen mit einem Katalysator zu 3-Chlorthiophen isomerisiert und dieses anschließend zwischen 40 und 120 °C zu 2.3-Dichlorthiophen chloriert, wobei das Verhältnis Chlor : 3-Chlorthiophen bei 0.3 : 1 bis 1.1 : 1 (mol/mol) liegt. Ein weiterer Gegenstand der Erfindung ist ein Verfahren zur Herstellung von 2.3-Dichlorthiophen, dadurch gekennzeichnet, daß man 3-Chlorthiophen zwischen 40 und 120 °C chloriert, wobei das Verhältnis Chlor : 3-Chlorthiophen bei 0.3 : 1 bis 1.1 : 1 (mol/mol) liegt.

Es war außerordentlich überraschend, daß die Chlorierung des 3-Chlorthiophens unter nahezu ausschließlicher Bildung des 2.3-Dichlorthiophens verläuft und daher dessen wirtschaftliche Herstellung ermöglicht.

Die Isomerisierung von 2-Chlorthiophen zu 3-Chlorthiophen wird bereits in der deutschen Patentanmeldung P 34 19 555.6 beschrieben. Dabei wird 2-Chlorthiophen zunächst allein oder zusammen mit einem organischen Verdünnungsmittel mit einem Isomerisierungskatalysator in Kontakt gebracht. Zur Verminderung des Einlaßpartialdrucks oder zur Standzeitverbesserung können dem Feed zusätzlich anorganische Gase wie $N_2$, Argon oder $H_2$ zugesetzt werden.

Als organische Verdünnungsmittel verwendet man im allgemeinen Benzol, Alkylbenzole oder mono- bzw. polyhalogenierte Benzole oder Toluole.

Das Molverhältnis des Verdünnungsmittels zu dem eingesetzten 2-Halogenthiophen liegt im allgemeinen bei Werten von 1 : 1 bis 5 : 1 (mol/mol).

Als Isomerisierungskatalysatoren eignen sich im allgemeinen natürliche und synthetische Zeolithe, vorzugsweise synthetische Zeolithe vom Faujasittyp, wie der Zeolith X (US-PS 2 882 244) und Zeolith Y (US-PS 3 130 007), Mordenittyp sowie Pentasiltyp, wie ZSM-5 (US-PS 3 702 886), ZSM-11 (US-PS 3 709 979), ZSM-8 (GB-PS 1 334 243), ZSM-12 (US-PS 3 832 449), ZSM-20 (US-PS 3 972 983), ZSM-21 (US-PS 4 046 859), ZSM-23 (US-PS 4 076 842), ZSM-35 (US-PS 4 016 245), ZSM-38 (US-PS 4 046 859), ZSM-48 (EP-A1-0 023 089). Besonders bevorzugt sind Zeolithe von Pentasiltyp. Das Si/Al-Verhältnis der Pentasile liegt vorzugsweise bei 20 bis 2000, das der Mordenite vorzugsweise bei 5 bis 100.

Als Zeolite für das erfindungsgemäße Verfahren sind auch solche geeignet, die zu den oben genannten Zeolithen strukturell analog sind, bei denen aber Aluminium beziehungsweise Silicium durch andere Gitteratome, wie Bor, Eisen, Gallium, Germanium, Titan oder Zirkon zumindest teilweise ersetzt ist.

Bei dem erfindungsgemäßen Verfahren werden die Zeolithe vorzugsweise in ihrer sauren Form eingesetzt. Die Herstellung der sauren Form erfolgt durch Ionenaustausch mit ein-, zwei- oder dreiwertigen Kationen, vorzugsweise mit $NH^+$, $H^+$, $Be^{2+}$, $Mg^{2+}$, $Ca^{2+}$, $La^{3+}$ oder Seltenerd-Kationen sowie mit Kombinationen dieser Kationen. Die so modifizierten Zeolithe werden außerdem vorzugsweise in der üblichen Art durch Calcinieren aktiviert, was eine weitere Art der Modifikation darstellt. Die Calcinierung wird vorzugsweise bei 350 bis 700 °C durchgeführt. Zur besseren Stabilisierung ist es vorteilhaft, die Calcinierung in Gegenwart von Wasserdampf, Ammoniak oder deren Mischungen bei Temperaturen zwischen 600 und 900 °C durchzuführen.

Die genannten Zeolithe werden für den technischen Einsatz mit Hilfe von Bindern in Strangform gebracht, wobei die Wahl des Binders die Selektivität und Lebensdauer beeinflußt.

Als Bindermaterial sind vor allem die Oxide, Hydroxide oder Hydroxychloride des Aluminiums und die Oxide des Siliciums, die Oxide des Titans und Zirkons sowie Tonmaterialien geeignet.

Die Isomerisierung kann sowohl in der Gasphase als auch in der Flüssigphase durchgeführt werden ; auch andere Verfahrensweisen, wie beispielsweise eine Reiselphase bei Anwendung eines höheren Druckes, sind denkbar.

Wird die erfindungsgemäße Isomerisierung in der Gasphase mit fest angeordnetem Katalysator

2

durchgeführt, so betragen die Reaktionstemperaturen im allgemeinen 150-550 °C, vorzugsweise 180-450 °C und die Drücke 1 bis 20 bar, vorzugsweise 2 bis 10 bar.

Bei der Flüssigphasen-Arbeitsweise wird die Isomerisierung bei Temperaturen von 150 bis 350 °C, vorzugsweise von 200 bis 300 °C und bei Drücken von 4 bis 100 bar, vorzugsweise 10 bis 50 bar, durchgeführt.

Die Belastung (WHSV = Weight-Hourly-Space-Velocity h$^{-1}$) sollte vorzugsweise zwischen 0,1 und 10 h$^{-1}$ liegen. Wird eine Regenerierung des Katalysators erforderlich, so kann sie durch kontrolliertes Abbrennen mit sauerstoffhaltigen Gasen erfolgen.

Die Isomerisierung verläuft sehr selektiv und ergibt in Abhängigkeit von Prozeßbedingungen, wie z. B. Temperatur und Verweilzeit sowie in Abhängigkeit vom Zeolithtyp hohe Anteile an 3-Chlorthiophen. Das erhaltene Isomerengemisch besteht aus 85 bis 90 Gew.-% 3-Chlorthiophen, 5 bis 10 Gew.-% 2-Chlorthiophen und geringen Anteilen an Thiophen. Dieses Isomerengemisch kann unmittelbar in der Chlorierungsstufe des erfindungsgemäßen Verfahrens eingesetzt werden (ggf. nach destillativer Abtrennung des Verdünnungsmittels). Man kann aber auch erst das 2-Chlorthiophen entfernen, ehe man chloriert.

Die Chlorierung wird in üblichen gußeisernen oder stählernen Rührwerkskesseln, die mit Mantel- und/oder Schlangenkühlung, Chloreinleitungs-, Abgas-, Füll- und Entleerungsrohren ausgestattet sind, mit gasförmigen Chlor durchgeführt. Auch Rieselphasenreaktoren, bei denen 3-Chlorthiophen am Kopf und gasförmiges Chlor im Mittelteil des Reaktors aufgegeben werden, sind zur kontinuierlichen Herstellung von 2.3-Dichlorthiophen bestens geeignet.

Das Verhältnis Chlor : 3-Chlorthiophen, ausgedrückt in mol pro mol, sollte bevorzugt bei 0,7 : 1 bis 0,95 : 1 liegen.

Die Chlorierung kann ohne zusätzliches Lösemittel in Substanz durchgeführt werden.

Die Chlorierungstemperaturen liegen bevorzugt zwischen 60 und 100 °C.

Das nach der Chlorierung des oben genannten Isomerengemisches aus 3- und 2-Chlorthiophen anfallende, im wesentlichen aus 2.3-Dichlorthiophen und geringen Anteilen an 2.5-Dichlorthiophen sowie aus Restanteilen von 2-Chlor- und 3-Chlorthiophen bestehende Produktgemisch wird destillativ aufgearbeitet.

Bei diskontinuierlicher Aufarbeitung wird zunächst das bei 128 °C bzw. 135 °C siedende 2-Chlor-/3-Chlorthiophen als Destillationsspitze abgenommen und danach die bei 162° und 173 °C siedenden 2.5- und 2.3-Dichlorthiophenisomeren getrennt. Ist das als Koppelprodukt anfallende 2.5-Dichlorthiophen unerwünscht, so kann nach der Isomerisierungsstufe mit einer aufwendigeren Destillation 2-Chlorthiophen von 3-Chlorthiophen getrennt werden. Die nachfolgende Chlorierung des 3-Chlorthiophens erbringt eine, auf 2-Chlorthiophen bezogene, 2.3-Dichlorthiophen-Ausbeute von über 80 %.

Die erfindung soll durch die nachfolgenden Beispiele erläutert werden, wobei diese aber in keiner Weise einschränkend sein sollen.


Beispiel 1 (Isomerisierung und anschließende Chlorierung des Isomerisats)

Ein gemäß US-Patentschrift 3 702 886 synthetisierter Zeolith ZSM-5 wurde zunächst 12 Stunden bei 550 °C thermisch vorbehandelt und danach im Verhältnis 2 : 1 mit Aluminiumoxid vermischt, mit einer verdünnten Mineralsäure angeteigt und zu 1.4 mm starken Extrudaten verarbeitet. Die erhaltenen Katalysatorstränge wurden anschließend bei 120 °C getrocknet und danach 2 Stunden bei 600 °C geglüht. Nach dieser Behandlung wurden die Stränge mehrmals mit einer 10 %igen Ammonsulfatlösung in Kontakt gebracht und die synthesebedingt vorhandenen Natriumionen gegen NH$_4$$^+$ erschöpfend ausgetauscht. Durch eine nochmalige thermische Behandlung dieser Stränge bei 600 °C erhielt man die saure H-Form des Zeolithen ZSM-5.

Zur Isomerisierung des 2-Chlorthiophens wurde ein Festbettreaktor von 20 mm Innendurchmesser und 1000 mm Länge mit 200 ml des oben beschriebenen Katalysators beladen und bei Atmosphärendruck und 300 °C mit 400 ml/h eines aus 2-Chlorthiophen und 1.2.4-Trichlorbenzol bestehenden Flüssigkeitsgemischs beschickt. Nach Durchsatz von 17,0 mol 2-Chlorthiophen wurde die Reaktion nach 15 Stunden unterbrochen und der kondensierte Reaktoraustrag aufgearbeitet. Hierzu wurde das Chlorthiophenisomerisat destillativ von 1.2.4-Trichlorbenzol getrennt und zur Chlorierung bereitgestellt.

Die zur Chlorierung verwendete Apparatur bestand aus einem 4 l-Dreihalskolben mit Rührer, Gaseinleitungsrohr, Innenthermometer und Rückflußkühler. Der durch den Rückflußkühler abgehende Chlorwasserstoff wurde durch Wasser oder Natronlauge absorbiert. Die Beheizung des Kolbens erfolgte durch ein Ölbad. Zur Durchführung der Chlorierung wurden 1 900 g aus der erwähnten destillativen Trennung erhaltenes Chlorthiophenisomerisat ohne Lösemittel in den Kolben vorgelegt.

Nachdem der Kolbeninhalt auf 90 °C temperiert war, wurde mit einer Chlordosierrate von 2 mol/h die Chlorierung gestartet. Im Verlauf der Chlorierung stieg hierbei die Kolbeninnentemperatur von 90 auf 105 °C an. Nach Einleiten von 16 mol Chlor wurde die Reaktion beendet und das Chlorthiophenisomerengemisch destilliert. Ein aus 2-/3-Chlorthiophen bestehender Vorlauf wurde zunächst am Kopf einer 60-bödigen Füllkörperkolonne abgenommen. Danach folgte nach einem kurzen Zwischenlauf 2.5-Dichlorthiophen mit geringen Anteilen an 2.3-Dichlorthiophen. Die unter Abreicherung von 2.5-Dichlorthiophen

3

verlaufende Destillation wurde bei Erreichen eines 2.3-Dichlorthiophengehalts von ca. 98 % im Kolonnensumpf beendet.

Die bei dieser Destillation gewonnenen 1 680 g an 2.3-Dichlorthiophen entsprechen einer auf 2-Chlorthiophen bezogenen Ausbeute von 65 %.

Nicht umgesetztes 2-Chlor- und 3-Chlorthiophen wurden zur erneuten Chlorierung oder gegebenenfalls zur erneuten Isomerisierung zurückgeführt. Die mit 2.3-Dichlorthiophen verunreinigte 2.5-Dichlorthiophenfraktion wurde separat zu 2.5-Dichlorthiophen aufgearbeitet.

**Beispiel 2 (Isomerisierung und anschließende Chlorierung des isolierten 3-Chlorthiophens)**

Unter Einsatz der im Beispiel 1 beschriebenen Apparatur wurden durch Isomerisierung von 2-Chlorthiophen 6.3 kg mit wenig Thiophen verunreinigtes Chlorthiopheniisomerisat erzeugt.

Das aus 85 Gew.-% 3-Chlorthiophen, 13 Gew.-% 2-Chlorthiophen und 2 Gew.-% Thiophen bestehende Isomerisat wurde an einer 80-bödigen Füllkörperkolonne bei Normaldruck in 3.4 kg 99 %iges 3-Chlorthiophen und 2.85 kg 2-/3-Chlorthiopheniisomerengemisch fraktioniert. Die bei der Fraktionierung anfallende 3-Chlorthiophenfraktion wurde in Substanz bei 90 °C in der im Beispiel 1 beschriebenen Apparatur mit einer Dosierrate von 3 mol/h chloriert. Der in Tabelle 1 dargestellte, durch gaschromatographische Analyse ermittelte Zusammenhang von 3-Chlorthiophenumsatz und 2.3-Dichlorthiophenselektivität gibt die außerordentlich hohe Selektivität der Chlorierung von 3-Chlorthiophen wieder.

| Umsatz an 3-Chlorthiophen (%) | Selektivität zu 2.3-Dichlorthiophen |
|---|---|
| 10 | 95 |
| 30 | 93 |
| 47 | 92 |
| 70 | 91 |
| 80 | 90 |
| 90 | 90 |

Tabelle 1 : Chlorierung von 3-Chlorthiophen

Die nach Beendigung der Chlorierung vorgenommene Aufarbeitung lieferte 3 530 g 2.3-Dichlorthiophen (entsprechend einer 80 %igen Ausbeute) und 340 g 3-Chlorthiophen, das zur erneuten Chlorierung eingesetzt werden kann.

**Patentansprüche**

1. Verfahren zur Herstellung von 2.3-Dichlorthiophen, dadurch gekennzeichnet, daß man 2-Chlorthiophen mit einem Katalysator zu 3-Chlorthiophen isomerisiert und dieses anschließend zwischen 40 und 120 °C zu 2.3-Dichlorthiophen chloriert, wobei das Verhältnis Chlor : 3-Chlorthiophen bei 0.3 : 1 bis 1.1 : 1 (mol/mol) liegt.

2. Verfahren nach Anspruch 1, dadurch gekennzeichnet, daß man als Katalysator einen sauren Zeolithen verwendet.

3. Verfahren nach Anspruch 1, dadurch gekennzeichnet, daß man als Katalysator einen Zeolithen vom Pentasiltyp verwendet.

4. Verfahren nach Anspruch 1, dadurch gekennzeichnet, daß man als Katalysator ein saures Pentasil verwendet.

5. Verfahren zur Herstellung von 2.3-Dichlorthiophen, dadurch gekennzeichnet, daß man 3-Chlorthiophen zwischen 40 und 120 °C chloriert, wobei das Verhältnis Chlor : 3-Chlorthiophen bei 0.3 : 1 bis 1.1 : 1 (mol/mol) liegt.

**Claims**

1. A process for preparing 2,3-dichlorothiophene, which comprises isomerizing 2-chlorothiophene with a catalyst to 3-chlorothiophene and subsequently chlorinating this 3-chlorothiophene between 40 and 120 °C to give 2,3-dichlorothiophene, the chlorine : 3-chlorothiophene ratio being 0.3 : 1 to 1.1 : 1 (mol/mol).

2. The process as claimed in claim 1, wherein the catalyst used is an acidic zeolite.

3. The process as claimed in claim 1, wherein the catalyst used is a zeolite of the pentasil type.

4. The process as claimed in claim 1, wherein the catalyst used is an acidic pentasil.

5. A process for preparing 2,3-dichlorothiophene, which comprises chlorinating 3-chlorothiophene between 40 an 120 °C, the chlorine : 3-chlorothiophene ratio being 0.3 : 1 to 1.1 : 1 (mol/mol).

**Revendications**

1. Procédé de préparation du 2,3-dichlorothiophène, procédé caractérisé en ce que l'on isomérise le 2-chlorothiophène en 3-chlorothiophène avec un catalyseur puis on chlore le 3-chlorothiophène entre 40 et 120 °C pour former le 2,3-dichlorothiophène, dans un rapport molaire du chlore au 3-chlorothiophène compris entre 0,3 et 1,1 (mol/mol).

2. Procédé selon la revendication 1 caractérisé en ce que l'on opère avec une zéolite acide comme catalyseur.

3. Procédé selon la revendication 1 caractérisé en ce que l'on opère avec une zéolite du genre pentasil comme catalyseur.

4. Procédé selon la revendication 1 caractérisé en ce que le catalyseur est un pentasil acide.

5. Procédé de préparation du 2,3-dichlorothiophène, caractérisé en ce que l'on chlore le 3-chlorothiophène entre 40 et 120 °C dans un rapport molaire du chlore au 3-chlorothiophène compris entre 0,3 et 1,1 (mol/mol).